# EUROPEAN PATENT SPECIFICATION

(11) **EP 3 916 738 B1**
(45) Date of publication and mention of the grant of the patent: **31.01.2024**
(21) Application number: 21150084.8
(22) Date of filing: 04.01.2021
(51) Int. Cl.: G16H 50/70, G16H 50/20

(54) **MEDICAL FACT VERIFICATION METHOD AND APPARATUS, ELECTRONIC DEVICE, AND STORAGE MEDIUM**
VERFAHREN UND GERÄT ZUR MEDIZINISCHEN FAKTENÜBERPRÜFUNG, ELEKTRONISCHE VORRICHTUNG UND SPEICHERMEDIUM
PROCÉDÉ ET APPAREIL DE VÉRIFICATION DE FAIT MÉDICAL, DISPOSITIF ÉLECTRONIQUE ET SUPPORT D'ENREGISTREMENT

(30) Priority: 29.05.2020 CN 202010473438
(43) Date of publication of application: 01.12.2021
(73) Proprietor: Beijing Baidu Netcom Science and Technology Co., Ltd., Beijing 100085 (CN)
(72) Inventor: FANG, Zhou, Beijing, 100085 (CN); SHI, Yabing, Beijing, 100085 (CN); JIANG, Ye, Beijing, 100085 (CN); CHAI, Chunguang, Beijing, 100085 (CN)
(74) Representative: Weickmann & Weickmann PartmbB

(56) References cited:
- US-A1- 2008 004 505
- US-A1- 2018 075 012
- US-A1- 2019 130 073

## Description

### TECHNICAL FIELD

The present application relates to the technical field of computers, in particular to the field of artificial intelligence. The present application can be applied to the field of knowledge graphs.

### BACKGROUND

The existing manners to verify a medical fact are mainly as follows: One is to verify it through manual searching and labeling, which requires a labeling personnel to have medical professional knowledge, but the labor cost is high, and it is difficult to process large-scale medical data. Another is to extract a fact occurring in a medical document by manually preconfiguring a text template or a part-of-speech template, and compare the extracted fact with the fact to be verified to complete the verification. However, the poor generalization of manually defined rules and consumption of labor cost make it difficult to process large-scale medical professional data.

US20180075012A1 discloses a cognitive natural language processing system. The cognitive natural language processing (NLP) system analyzes a portion of natural language text to identify an attribute specified in the natural language text. The cognitive NLP system analyzes the portion of natural language text to determine whether a known negation trigger is present in the natural language text in association with the attribute.

US20080004505A1 discloses a vascular interventional radiology procedure using a combination of natural language processing (NLP) and human medical coders. Medical billing codes are efficiently extracted from medical reports using a NLP engine and a graphical user interface optimized for understanding VasIP medical procedure.

US20190130073A1 discloses a system for automatically processing text comprising information regarding a patient encounter to assign medical codes to the text is provided.

### SUMMARY

In order to solve at least one problem in the existing technology, a medical fact verification method and apparatus, and a storage medium are provided according to embodiments of the application.

The present disclosure is set out in the appended set of claims.

One embodiment of the above application has the following advantages or benefits: by using the attribute decision model and the relevancy decision model, the attribute and the relevancy decisions are sequentially completed, so that the medical fact can be determined to be correct in a case that the attribute described by the candidate evidence accords with the target attribute and the relevancy accords with a certain condition, which solves the technical problem of high cost caused by manual verification in the existing technology, thereby reducing the labor cost; and being more suitable for large-scale data processing.

Other effects of the above alternatives will be described below in connection with specific embodiments.

### BRIEF DESCRIPTION OF THE DRAWINGS

The drawings are included to provide a better understanding of the solution and are not to be construed as limiting the present application, wherein:
FIG. 1 shows a flowchart I of a medical fact verification method according to an embodiment of the present application;
FIG. 2 shows a flowchart II of a medical fact verification method according to an embodiment of the present application;
FIG. 3 shows a schematic diagram of an attribute decision model according to an embodiment of the present application;
FIG. 4 shows a schematic diagram of a relevancy decision model according to an embodiment of the present application;
FIG. 5 shows a structural diagram I of a medical fact verification apparatus according to an embodiment of the present application;
FIG. 6 shows a structural diagram II of a medical fact verification apparatus according to an embodiment of the present application;
FIG. 7 shows a structural diagram III of a medical fact verification apparatus according to an embodiment of the present application;
FIG. 8 shows a structural diagram IV of a medical fact verification apparatus according to an embodiment of the present application;
FIG. 9 shows a structural diagram V of a medical fact verification apparatus according to an embodiment of the present application; and
FIG. 10 shows a block diagram of an electronic device used to implement a medical fact verification method of an embodiment of the present application.

### DETAILED DESCRIPTION

The exemplary embodiments of the application will be described below in combination with drawings, including various details of the embodiments of the application to facilitate understanding, which should be considered as exemplary only. Therefore, those of ordinary skill in the art should realize that various changes and modifications can be made to the embodiments described herein without departing from the scope of the present application. Likewise, descriptions of well-known functions and structures are omitted in the following description for clarity and conciseness.

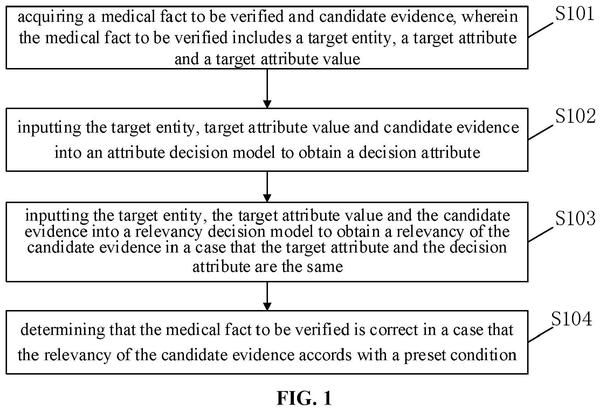

The embodiment of the present application provides A medical fact verification method is provided according to an embodiment of the present application, which can be applied to an electronic device, and the electronic device can have data processing functions such as numerical calculation, logic calculation, and data storage. Referring to FIG. 1, a flowchart of a medical fact verification method is shown, the method includes:
S101, acquiring a medical fact to be verified and candidate evidence, wherein the medical fact to be verified includes a target entity, a target attribute and a target attribute value;
S 102, inputting the target entity, target attribute value and candidate evidence into an attribute decision model to obtain a decision attribute;
S103, inputting the target entity, the target attribute value and the candidate evidence into a relevancy decision model to obtain a relevancy of the candidate evidence in a case that the target attribute and the decision attribute are the same, and
S104, determining that the medical fact to be verified is correct in a case that the relevancy of the candidate evidence accords with a preset condition.

In an embodiment of the present application, each medical fact may be represented in the form of an SPO triplet, S representing an entity, P representing an attribute, and O representing an attribute value. Taking a medical fact <measles, symptoms, skin maculopapules> as an example, the entity S is measles, the attribute P is symptoms, and the attribute value O is skin maculopapules.

Correspondingly, the processing of S101-S103 may be configured for processing the medical fact to be verified at this time, and may be configured for processing different medical facts to be verified at different times. An entity, an attribute and an attribute value in each medical fact to be verified are correspondingly referred to as a target entity, a target attribute and a target attribute value in the present application.

Alternatively, the attribute in the medical fact includes at least one of a clinical feature, an etiology, a pathology, a therapeutic regimen, a recommended medication, a complication, and a drug effect.

Alternatively, the candidate evidence is candidate evidence that verifies whether the medical fact is correct, and the candidate evidence is retrieved from a designated medical database based on the medical fact to be verified. The designated medical database may store various types of authoritative medical materials, including books, magazines, papers, etc.

The embodiment can be configured for constructing a medical knowledge graph. In the process of constructing the medical knowledge graph, a medical fact such as <measles, symptoms, skin maculopapules> are extracted by a machine, and candidate evidence can be retrieved from a designated medical document library according to the medical fact to be verified. The verification of the medical fact is completed through the verification method provided by S101-S104, if the verification is correct, the medical fact is formally determined to be added into the medical knowledge graph, meanwhile, the relevancy of the candidate evidence can be configured for determining the corresponding supporting evidence, which is conducive to improving the accuracy of medical graph data.

In the embodiment, for the medical fact to be verified and the candidate evidence, firstly, an attribute corresponding to a target entity and a target attribute value described by the candidate evidence are decided through an attribute decision model to obtain a decision attribute; if the decision attribute accords with the target attribute, the relevancy of the candidate evidence with respect to the target entity and the target attribute value is decided through a relevancy decision model; and when the relevancy of the candidate evidence accords with a condition, the medical fact is verified to be correct.

According to the embodiment of the application, through the attribute decision model and the relevancy decision model, a dual decision of the attribute and relevancy decision can be completed; the medical fact can be verified to be correct in a case that the attribute described by the candidate evidence accords with the target attribute and the relevancy accords with the condition, which can strengthen the correlation decision of the medical fact and the candidate evidence, improve the stringency of the verification result, and better meet the requirements of medical professional data processing; moreover, manual labeling or manual defined rules are not needed, reducing labor cost, and more suitable for large-scale data processing.

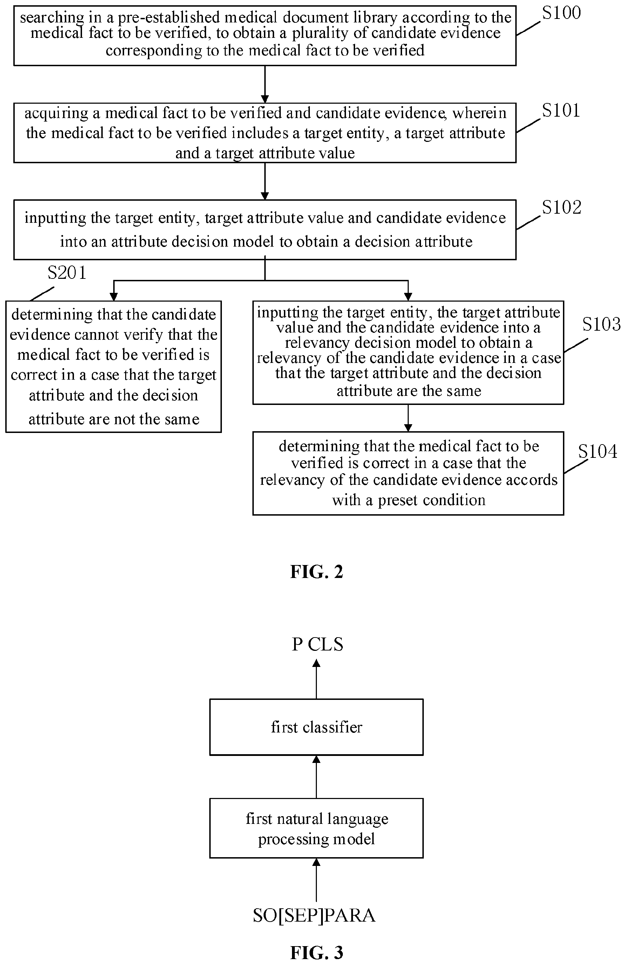

In an embodiment, referring to FIG. 2, prior to S101, the method further includes: S 100, searching in a pre-established medical document library according to the medical fact to be verified, to obtain a plurality of candidate evidence corresponding to the medical fact to be verified.

In an embodiment, referring to FIG. 2, after S102, the method further includes: S201, determining that the candidate evidence cannot verify that the medical fact to be verified is correct in a case that the target attribute and the decision attribute are not the same. For example, in the case where the medical fact to be verified is <measles, symptoms, skin maculopapules>, the decision attribute obtained in S 102 based on certain candidate evidence is "therapeutic regimens", which is different from the target attribute "symptom", at which time it is determined that the candidate evidence cannot verify that the medical fact to be verified is correct.

According to the embodiment, when the attribute decision model decides that the attribute does not accord, it is decided that the candidate evidence cannot verify that the medical fact to be verified is correct, and the verification of the current candidate evidence is stopped, which effectively improves the calculation efficiency; and remarkably improves the verification efficiency especially in processing large-scale medical professional data.

In an embodiment, referring to FIG. 3, a schematic diagram of an attribute decision model adopted in S 102 is shown, the attribute decision model includes a first natural language processing model and a first classifier.

S102, inputting the target entity, the target attribute value and the candidate evidence into an attribute decision model to obtain a decision attribute, includes:
inputting the target entity, the target attribute value and the candidate evidence into the first natural language processing model to obtain a first feature vector of the target entity, the target attribute value and the candidate evidence; and
inputting the first feature vector into the first classifier to obtain the decision attribute.

According to the embodiment, the attribute decision model adopts a structure with a natural language processing model and a classifier. Features are extracted from the entity, the attribute value and the candidate evidence firstly, then classification is performed on the basis of the features so as to decide the attribute to which they belong. The structure is simple, and the attribute decision can be realized.

The structure of the attribute decision model given by the above-mentioned embodiment is an alternative mode, and in other embodiments, a person skilled in the art could also realize the embodiments of deciding attributes based on target entities, target attribute values and candidate evidence through a structure with other models within the scope of the embodiment of the present application.

Optionally, the first natural language processing model adopts an enhanced representation from knowledge integration (ERNIE). In other alternatives, a BERT model may be used as the first natural language processing model.

Optionally, the first classifier adopts a Softmax classifier. It is also within the scope of the embodiments of the present application that other classifiers are selected to complete the same implementation of processing the analyzed feature vector for classification based on the natural language processing model to determine the corresponding attributes.

Alternatively, referring to FIG. 3, the target entity S, the target attribute value O, and the candidate evidence PARA are input into the attribute decision model in the form of "SO[SEP]PARA" in S102, SEP being a separator. In addition, "P CLS" in FIG. 3 represents the attribute P output, and "CLS" represents output. Taking the medical fact <measles, symptoms, skin maculopapules> to be verified and the candidate evidence "XXXXX" as an example, "measles skin maculopapules [SEP] XXXXX" is input to the attribute decision model, and the attribute decision model decides the attribute "symptoms" based on the output.

In an embodiment, the attribute decision model adopted in S102 is established by:
constructing the attribute decision model by using the first natural language processing model and the first classifier, wherein the first natural language processing model is a natural language processing model obtained through pre-training based on a medical corpus; and
training the constructed attribute decision model using a plurality of first sample data, each first sample data including a correct medical fact and supporting evidence.

In the embodiment, the first natural language processing model that is pre-trained through the medical corpus is adopted, and the training of the attribute decision model can be realized through fine adjustment, namely a small amount of sample data is adopted for training, which greatly reduces the quantity requirement on the sample data, and reduces the cost of labeling the sample data manually.

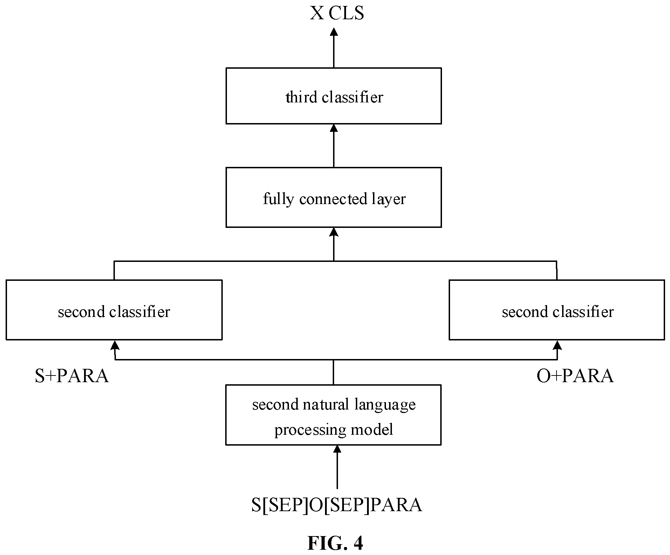

In an embodiment, referring to FIG. 4, a schematic diagram of the attribute decision model adopted in S103 is shown, the relevancy decision model includes a second natural language processing model, two second classifiers, a fully connected layers (FC) and a third classifier;

Correspondingly, the inputting the target entity, the target attribute value and the candidate evidence into the relevancy decision model to obtain a relevancy of the candidate evidence in S103 includes:
inputting the target entity, the target attribute value and the candidate evidence into the second natural language processing model to obtain a first layer feature vector of the target entity and the candidate evidence and a first layer feature vector of the target attribute value and the candidate evidence;
inputting the first layer feature vector of the target entity and the candidate evidence and the first layer feature vector of the target attribute value and the candidate evidence into the two second classifiers respectively, to obtain a second layer feature vector of the target entity and the candidate evidence and a second layer feature vector of the target attribute value and the candidate evidence; and
inputting the second layer feature vector of the target entity and the candidate evidence and the second layer feature vector of the target attribute value and the candidate evidence, which have been subjected to processing of the fully connected layer, into the third classifier to obtain the relevancy of the candidate evidence.

According to the embodiment, on the basis of adopting the natural language processing model and the classifier, the data output by the natural language processing model is split into the feature vector of the entity and the candidate evidence, and the feature vector of the attribute value and the candidate evidence, which are then processed by the two classifiers, respectively, thereby effectively strengthening the association between the candidate evidence and each of the entity and attribute value, and improving the accuracy of the relevancy.

The neurons of the output layer of the fully connected layer are connected to each neuron of the input layer. Therefore, by using the fully connected layer, the second layer feature vector of the target entity and the candidate evidence and the second layer feature vector of the target attribute value and the candidate evidence can be processed into a column item vector, facilitating the subsequent processing of the third classifier.

Optionally, the second natural language processing model adopts an ERNIE model. In other alternatives, a BERT model may be used as the first natural language processing model.

Alternatively, the second classifier and the third classifier each may adopt a Softmax classifier.

Alternatively, referring to FIG. 4, the target entity S, the target attribute value O, and the candidate evidence PARA are input into the relevancy decision model in the form of "S[SEP]O[SEP]PARA" in S103. Taking the medical fact <measles, symptoms, skin maculopapules> to be verified and the candidate evidence "XXXXX" as an example, "measles[SEP]skin maculopapules[SEP]XXXXX" is input the relevancy decision model.

In addition, "X CLS" in FIG. 4 represents X output, and X is the relevancy of the candidate evidence.

In an embodiment, the attribute decision model adopted in S103 is established by:
constructing the relevancy decision model by using the second natural language processing model, the two second classifiers, the fully connected layer and the third classifier, wherein the second natural language processing model is a natural language processing model obtained through pre-training based on a medical corpus; and
training the constructed relevancy decision model by using a plurality of second sample data, wherein each second sample data includes a medical fact, supporting evidence and a relevancy of the medical fact and the supporting evidence.

In the embodiment, the second natural language processing model that is pre-trained through the medical corpus is adopted, and the training of the relevancy decision model can be realized through fine adjustment, namely a small amount of sample data is adopted for training, which greatly reduces the quantity requirement on the sample data, and reduces the cost of labeling the sample data manually.

Alternatively, the second sample data may be obtained from known SPO triples in an existing medical knowledge base and results returned by an evidence retrieval module.

Alternatively, in the second sample data, the relevancy of the medical fact and the supporting evidence may be manually labeled.

In the embodiment, the second natural language processing model that is pre-trained through the medical corpus is adopted, and the training of the relevancy decision model can be realized through fine adjustment, namely a small amount of sample data is adopted for training, which greatly reduces the quantity requirement on the sample data, and reduces the cost of labeling the sample data manually.

In one example, the relevancy of the candidate evidence output by the relevancy decision model of S103 may be a numerical value, such as any number of interval [0, 1]. The greater the relevancy of the candidate evidence, the higher the relevancy of the candidate evidence, indicating that the candidate evidence can support the correctness of the medical fact, and the higher the probability that the medical fact is correct from the side.

Compared with other industries, the medical industry has stricter and more rigorous requirements on the overall data accuracy rate. Therefore, the attribute decision model and the relevancy decision model provided by the embodiment are ingenious in model structure, improving the accuracy rate of the verification result, and meeting the strict requirements of the medical industry on data. Moreover, according to the model provided by the embodiment of the application, through the basic features, a suitable deep learning model structure designed and the training on large-scale labeled data, high accuracy and recall rate can be obtained without depending on high-level features defined manually, and labor cost is reduced.

In an embodiment, S 104 includes:
in a case that the relevancy of at least one candidate evidence in a plurality of candidate evidence is greater than a preset threshold value, determining that the medical fact to be verified is correct, and taking the candidate evidence with a highest relevancy in the at least one candidate evidence as supporting evidence for determining that the medical fact is correct.

After being verified by the attribute decision model, the correctness of the medical fact can be verified if the relevancy is greater than the preset value. The decision is simple and the accuracy is high. Meanwhile, the candidate evidence with the highest relevancy is selected as the supporting evidence to provide a basis for verifying the correctness of the medical fact.

With regard to the above-mentioned S 104, it is to be explained that if the relevancy of only one candidate evidence among a plurality of candidate evidence is greater than a preset threshold value, this candidate evidence with the relevancy greater than the preset value is directly considered to be the candidate evidence with the highest relevancy. In addition, if the medical fact only corresponds to one candidate evidence, if the relevancy of the candidate evidence is greater than a preset threshold value, the medical fact to be verified is verified to be correct, and the candidate evidence with the highest relevancy is used as the supporting evidence for determining that the medical fact is correct.

In other embodiments, the preset condition in S 104 can also be set as other conditions, for example, the relevancy of candidate evidence exceeding a preset number is set to be greater than a preset threshold value, and the value of the preset number is greater than 1; for another example, the percentage of candidate evidence with a relevancy greater than a predetermined threshold among the plurality of candidate evidence is greater than a predetermined percentage.

In other embodiments, S 104 may alternatively include selecting a plurality of candidate evidence whose relevancy ranking precedes as supporting evidence, and presenting the plurality of supporting evidence according to the relevancy ranking.

In an embodiment, the method of this embodiment further includes: if there is no relevancy of at least one candidate evidence being greater than the preset threshold value, determining that the medical fact is incorrect. No relevancy of at least one candidate evidence being greater than the preset threshold value includes the relevancy of each of the candidate evidence being less than the preset threshold value and the candidate evidence having no corresponding relevancy (i.e. the decision attributes obtained in S102 are all different from the target attributes).

The above S101-S104 are described in detail below by way of an example:
In S101, a medical fact to be verified and candidate evidence are obtained, wherein
the medical fact to be verified is <measles, symptoms, skin maculopapules>,
target entity: "measles",
target attribute: "symptoms", and
target attribute value: "skin maculopapules".

The candidate evidence is that "measles is a viral infectious disease caused by measles virus, and belongs to Category B infectious disease among the notifiable infectious diseases in China. The main clinical manifestations of measles include fever, cough, runny nose and other catarrhal symptoms and conjunctivitis, and the characteristic manifestations of measles are Koplik spots and skin maculopapules".

In S102, the target entity "measles", the target attribute value "skin maculopapules", and the candidate evidence are put into the attribute decision model to obtain a decision attribute "symptoms" corresponding to the "measles" and the "skin maculopapules".

Specifically, referring to FIG. 3, the attribute decision model includes the first natural language processing model and the first classifier. The first feature vector of "measles", "skin maculopapules" and the candidate evidence are extracted through the first natural language processing model, and then the attribute is determined to be "symptom" through the first classifier according to the first feature vector.

In S103, because the target attribute "symptom" and the decision attribute "symptom" are the same, the target entity "measles" and the target attribute value "skin maculopapules" are input into the relevancy decision model to obtain a relevancy of the candidate evidence with respect to the target entity "measles" and the target attribute value "skin maculopapules", and the relevancy of the candidate evidence is assumed to be 0.8.

Specifically, referring to FIG. 4, the relevancy decision model includes a second natural language processing model, two second classifiers, a fully connected layer, and a third classifier. Firstly, a first layer feature vector of "measles" and the candidate evidence, and a first layer feature vector of "skin maculopapules" and the candidate evidence are obtained through the second natural language processing model; secondly, a second layer feature vector of "measles" and the candidate evidence, and a second layer feature vector of "skin maculopapules" and the candidate evidence are obtained correspondingly through the two second classifiers according to the first layer feature vector of "measles" and the candidate evidence and the first layer feature vector of "skin maculopapules" and candidate evidence, respectively; and thirdly, the second layer feature vector of "measles" and the candidate evidence and the second layer feature vector of "skin maculopapules" and the candidate evidence are input to the third classifier after being processed through the fully connected layer, to obtain the relevancy of the candidate evidence to be output by the third classifier.

In S104, assuming that the preset condition is that the relevancy is greater than 0.7, and since 0.8>0.7, the relevancy 0.8 of the candidate evidence accords with the preset condition and the medical fact <measles, symptoms and skin maculopapules> to be verified is determined to be correct, and the candidate evidence can be used as supporting evidence for determining that <measles, symptoms and skin maculopapules> is correct.

An example of the verification process of one candidate evidence is given above. For the case where there are multiple candidate evidence, such as candidate evidence A, candidate evidence B, and candidate evidence C, similarly, the relevancies of candidate evidence A, candidate evidence B and candidate evidence C can be solved by S101-step S104, and the relevancies obtained are 0.3, 0.75, 0.8 in order. Because there is candidate evidence with a relevancy greater than 0.7, the medical fact can be verified to be tenable, and meanwhile, candidate evidence C with the highest relevancy can be selected to serve as the supporting evidence.

The following is an example of an output medical fact verification result, specifically:
"S": "measles",
"P": "symptoms",
"O": "skin maculopapules",
"label": "1",
"evidence": "section V Measles.

Measles is a viral infectious disease caused by measles virus, and belongs to Category B infectious disease among the notifiable infectious diseases in China. The main clinical manifestations of measles include fever, cough, runny nose and other catarrhal symptoms and conjunctivitis, and the characteristic manifestations of measles are Koplik spots and skin maculopapules".

Label indicates the verification result of the medical fact, label = 1 indicates that the verification result is correct, and label = 0 indicates that the verification result is wrong; and evidence represents supporting evidence determining that the medical fact is correct. Therefore, in the above example, the verification result is correct for the medical fact SPO <measles, symptoms, skin maculopapules> to be verified, and the above-mentioned evidence field is selected from the 8th edition of Infectious Diseases as the supporting evidence for determining that the medical fact is correct.

The method realized by the embodiment of the present application is a medical fact verification method based on a pre-training language model, and effectively improves the effect problem of fact verification on medical data. The method provided by the embodiment of the present application has at least one of the following advantages:
1. it has strong versatility and can deal with a large and wide range of medical fact verification issues;
2. the labor cost is low, mainly embodied in two aspects: firstly, for a new fact type, a new document set and a new expression mode, an extraction rule does not need to be redefined manually, and a correct result can be given according to the generalization of the model itself; secondly, the model is established in a mode of combining pre-training and fine adjustment, which reduces the requirements for the number of labeled samples, thereby reducing the cost of manually labeled samples; and
3. compared with a general fact verification method, the embodiment of the present application can be suitable for medical fact verification, and has strict data requirements, bringing certain effect improvement on medical data.

Correspondingly, the embodiment of the present application also provides a medical fact verification apparatus, and the included various modules thereof can be carried or arranged in the hardware of the electronic device, for example, the memory of the computer can carry the various modules of the device, to enable the central processing unit (CPU) of the computer to run the various modules in the memory.

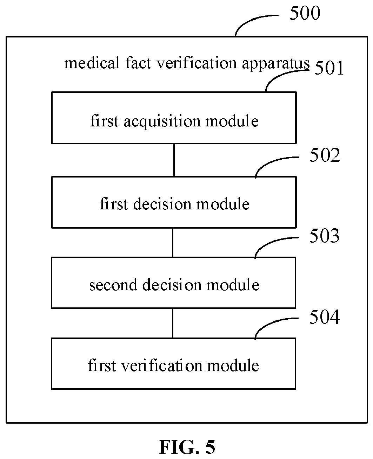

Referring to FIG. 5, a schematic diagram of a medical fact verification apparatus 500 is shown, and the apparatus 500 includes:
a first acquisition module 501 configured for acquiring a medical fact to be verified and candidate evidence, wherein the medical fact to be verified includes a target entity, a target attribute and a target attribute value;
a first decision module 502 configured for inputting the target entity, the target attribute value and the candidate evidence into an attribute decision model to obtain a decision attribute;
a second decision module 503 configured for inputting the target entity, the target attribute value and the candidate evidence into a relevancy decision model to obtain a relevancy of the candidate evidence in a case that the target attribute and the decision attribute are the same; and
a first verification module 504 configured for determining that the medical fact to be verified is correct in a case that the relevancy of the candidate evidence accords with a preset condition.

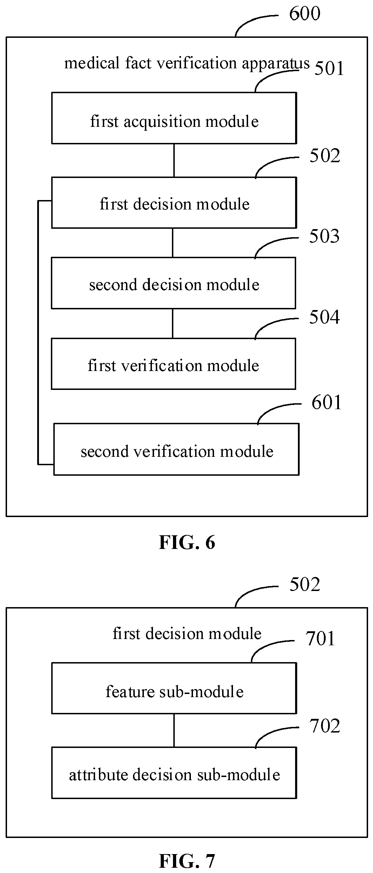

In an embodiment, referring to FIG. 6, a medical fact verification apparatus 600 further includes: a second verification module 601 configured for determining that the candidate evidence cannot verify that the medical fact to be verified is correct if the target attribute and the decision attribute are not the same.

In an embodiment, the attribute decision model includes a first natural language processing model and a first classifier.

Referring to FIG. 7, the first decision module 502 includes:
a feature sub-module 701 configured for inputting the target entity, the target attribute value and the candidate evidence into the first natural language processing model to obtain a first feature vector of the target entity, the target attribute value and the candidate evidence; and
an attribute decision sub-module 702 configured for inputting the first feature vector into the first classifier to obtain the decision attribute.

In an embodiment, the attribute decision model is established by:
constructing the attribute decision model by using the first natural language processing model and the first classifier, wherein the first natural language processing model is a natural language processing model obtained through pre-training based on a medical corpus; and
training the constructed attribute decision model by using a plurality of first sample data, each first sample data including a correct medical fact and supporting evidence.

In an embodiment, the relevancy decision model includes a second natural language processing model, two second classifiers, a fully connected layer, and a third classifier;

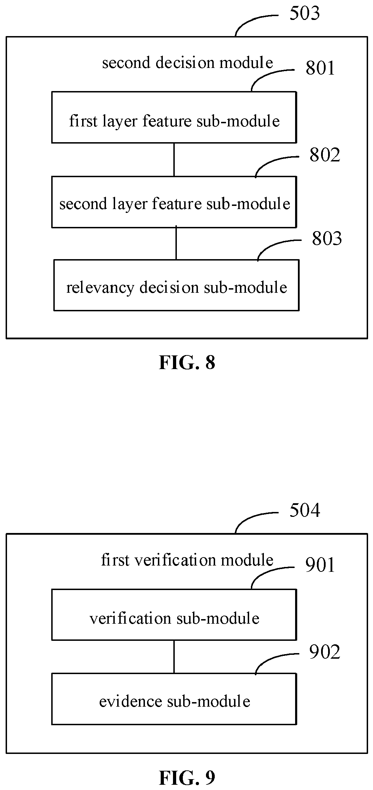

Referring to FIG. 8, the second decision module 503 includes:
a first layer feature sub-module 801 configured for inputting the target entity, the target attribute value and the candidate evidence into the second natural language processing model to obtain a first layer feature vector of the target entity and the candidate evidence and a first layer feature vector of the target attribute value and the candidate evidence;
a second layer feature sub-module 802 configured for inputting the first layer feature vector of the target entity and the candidate evidence and the first layer feature vector of the target attribute value and the candidate evidence into the two second classifiers respectively, to obtain a second layer feature vector of the target entity and the candidate evidence and a second layer feature vector of the target attribute value and the candidate evidence; and
a relevancy decision sub-module 803 configured for inputting the second layer feature vector of the target entity and the candidate evidence and the second layer feature vector of the target attribute value and the candidate evidence, which have been subjected to processing of the fully connected layer, into the third classifier to obtain the relevancy of the candidate evidence.

In an embodiment, the relevancy decision model is established by:
constructing the relevancy decision model by using the second natural language processing model, the two second classifiers, the fully connected layer and the third classifier, wherein the second natural language processing model is a natural language processing model obtained through pre-training based on a medical corpus; and
training the constructed relevancy decision model by using a plurality of second sample data, wherein each second sample data includes a medical fact, supporting evidence and a relevancy of the medical fact and the supporting evidence.

In an embodiment, referring to FIG. 9, the first verification module 504 includes:
a verification sub-module 901 configured for determining that the medical fact to be verified is correct in a case that the relevancy of at least one candidate evidence in a plurality of candidate evidence is greater than a preset threshold value; and
an evidence sub-module 902 configured for taking the candidate evidence with the highest relevancy among the at least one candidate evidence as supporting evidence for determining that the medical fact is correct.

For the functions of the modules in the apparatus in the embodiments of the present application, reference may be made to the corresponding descriptions in the foregoing method, and details are not described herein again.

An electronic device and a readable storage medium are provided according to embodiments of the application.

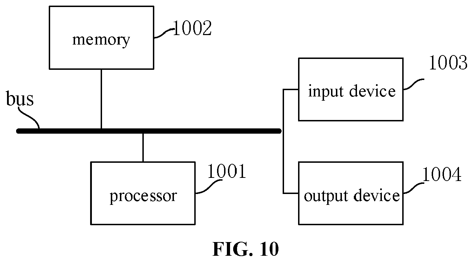

As shown in FIG. 10, a block diagram of an electronic device for a medical fact verification method according to an embodiment of the present application is shown. The electronic device is intended to represent various forms of digital computers, such as laptop computers, desktop computers, workstations, personal digital assistants, servers, blade servers, mainframe computers, and other suitable computers. The electronic device may also represent various forms of mobile devices, such as personal digital processing, cellular telephones, smart phones, wearables, and other similar computing devices. The components shown herein, their connections and relationships, and their functions are by way of example only and are not intended to limit the implementations of the present application described and/or claimed herein.

As shown in FIG. 10, the electronic device includes: one or more processors 1001, a memory 1002, and interfaces for connecting components, including a high-speed interface and a low-speed interface. The various components are interconnected using different buses and may be mounted on a common motherboard or otherwise as desired. The processor may process instructions executed in the electronic device, including instructions stored in or on the memory to display graphical information of the GUI on an external input/output device (such as a display device coupled to an interface). In other embodiments, multiple processors and/or multiple buses may be used with multiple memories, if desired. Also, multiple electronic devices may be connected, each providing some of the necessary operations (e.g., as an array of servers, a set of blade servers, or a multiprocessor system). One processor 1001 is taken as an example in FIG. 10.

The memory 1002 is a non-transitory computer-readable storage medium provided herein. Wherein the memory stores instructions executable by at least one processor to cause the at least one processor to perform the medical fact verification method provided herein. The non-transitory computer-readable storage medium of the present application stores computer instructions for enabling a computer to perform the medical fact verification method provided herein.

The memory 1002, as a non-transitory computer-readable storage medium, may be used to store non-transitory software programs, non-transitory computer-executable programs, and modules, e.g. program instructions/modules corresponding to methods for medical fact verification in embodiments of the present application (such as the first acquisition module 501, the first decision module 502, the second decision module 503, and the second decision module 504 shown in FIG. 5). The processor 1001 executes the various functional applications of the server and the data processing, i.e. implement the medical fact verification method in the above-described method embodiments, by running non-transient software programs, instructions and modules stored in the memory 1002.

The memory 1002 may include a storage program area and a storage data area, wherein the storage program area may store an operating system, an application program required for at least one function; and the storage data area may store data created according to use of the electronic device for the medical fact verification method, etc. In addition, the memory 1002 may include a high speed random access memory, and may also include a non-transitory memory, such as at least one disk storage device, flash memory device, or other non-transitory solid state storage device. In some embodiments, the memory 1002 optionally includes memories remotely located with respect to the processor 1001, which may be connected via a network to the electronic device for the medical fact verification method. Examples of such networks include, but are not limited to, the Internet, intranets, local area networks, mobile communication networks, and combinations thereof.

The electronic device may further include: an input device 1003 and an output device 1004. The processor 1001, the memory 1002, the input device 1003, and the output device 1004 may be connected by a bus or otherwise, as exemplified in FIG. 10 by a bus connection.

The input device 1003 may receive input numeric or character information and generate key signal inputs related to user settings and functional controls of an electronic device for medical fact verification, such as touch screens, keypads, mice, track pads, touch pads, pointing sticks, one or more mouse buttons, track balls, joysticks, and other input devices. The output device 1004 may include a display apparatus, an auxiliary lighting device (e.g., LED), and a tactile feedback device (e.g., vibration motor), etc. The display apparatus may include, but is not limited to, a liquid crystal display (LCD), a light emitting diode (LED) display, and a plasma display. In some embodiments, the display apparatus may be a touch screen.

Various embodiments of the systems and techniques described herein may be implemented in digital electronic circuitries, integrated circuit systems, application specific ASICs (application specific integrated circuits), computer hardware, firmware, software, and/or combinations thereof. These various embodiments may include: implementing in one or more computer programs, which can be executed and/or interpreted on a programmable system including at least one programmable processor, which can be a dedicated or general-purpose programmable processor capable of receiving data and instructions from, and transmit data and instructions to, a memory system, at least one input device, and at least one output device.

These computing programs (also referred to as programs, software, software applications, or codes) include machine instructions of programmable processors, and may be implemented using high-level procedural and/or object-oriented programming languages, and/or assembly/machine languages. As used herein, the terms "machine-readable medium" and "computer-readable medium" refer to any computer program product, apparatus, and/or device (e.g., magnetic disk, optical disk, memory, programmable logic device (PLD)) for providing machine instructions and/or data to a programmable processor, including a machine-readable medium that receives machine instructions as machine-readable signals. The term "machine-readable signal" refers to any signal used to provide machine instructions and/or data to a programmable processor.

To provide interaction with a user, the systems and techniques described herein may be implemented on a computer having: a display device (e.g., a CRT (cathode ray tube) or LCD (liquid crystal display) monitor) for displaying information to the user; and a keyboard and a pointing device (e.g., a mouse or a trackball) through which a user can provide input to the computer. Other types of devices may also be used to provide interaction with the user; for example, the feedback provided to the user may be any form of sensory feedback (e.g., visual feedback, auditory feedback, or tactile feedback); and input from the user may be received in any form (including acoustic input, voice input, or tactile input).

The systems and techniques described herein may be implemented in a computing system that includes a background component (e.g., as a data server), or a computing system that includes a middleware component (e.g., an application server), or a computing system that includes a front-end component (e.g., a user computer having a graphical user interface or a web browser through which the user may interact with embodiments of the systems and techniques described herein), or in a computing system that includes any combination of such background component, middleware component, or front-end component. The components of the system may be interconnected by any form or medium of digital data communication (e.g., a communication network). Examples of communication networks include: Local Area Networks (LANs), Wide Area Networks (WANs), and the Internet.

The computer system may include a client and a server. The client and server are typically remote from each other and typically interact through the communication network. The relationship between the client and the server is generated by computer programs running on the corresponding computers and having a client-server relationship with each other.

According to the technical scheme of the embodiment of the application, by the adoption of the attribute decision model and the relevancy decision model, the attribute and the relevancy decisions are sequentially completed, so that the correct technical means for verifying the medical fact can be realized in the case that the attribute described by the candidate evidence accords with the target attribute and the relevancy accords with the condition, solving the technical problem of high cost caused by manual verification in the existing technology, and reducing the labor cost; and the method is more suitable for large-scale data processing.

It will be appreciated that the various forms of flows, reordering, adding or removing steps shown above may be used. For example, the steps recited in the present application may be performed in parallel, sequentially or may be performed in a different order, so long as the desired results of the technical solutions disclosed in the present application can be achieved, and no limitation is made herein.

The above description only relates to specific embodiments of the present application, but the scope of protection of the present application is not limited thereto, and any of those skilled in the art can readily contemplate various changes or replacements within the technical scope of the present application. All these changes or replacements should be covered by the scope of protection of the present application. Therefore, the scope of protection of the present application should be determined by the scope of the appended claims.

The present application discloses a medical fact verification method and apparatus, an electronic device, and a storage medium, and relates to the field of computer technologies, in particular to the field of artificial intelligences. The present application can be applied to the field of knowledge graphs. The specific implementation scheme is as follows: acquiring a medical fact to be verified and candidate evidence, wherein the medical fact to be verified includes a target entity, a target attribute and a target attribute value; inputting the target entity, the target attribute value and the candidate evidence into an attribute decision model to obtain a decision attribute; inputting the target entity, the target attribute value and the candidate evidence into a relevancy decision model to obtain a relevancy of the candidate evidence in a case that the target attribute and the decision attribute are the same; and determining that the medical fact to be verified is correct in a case that the relevancy of the candidate evidence accords with a preset condition. The present application is beneficial to reduction of labor cost.

## Claims

1. A medical fact verification method, performed by a computer, the method comprising:
acquiring (S101) a medical fact to be verified and candidate evidence, wherein the medical fact to be verified comprises a target entity, a target attribute and a target attribute value, wherein the target attribute comprises at least one of a clinical feature, an etiology, a pathology, a therapeutic regimen, a recommended medication, a complication, and a drug effect, and the candidate evidence is retrieved from a designated medical database based on the medical fact to be verified;
inputting (S102) the target entity, the target attribute value and the candidate evidence into an attribute decision model to obtain a decision attribute;
inputting (S103) the target entity, the target attribute value and the candidate evidence into a relevancy decision model to obtain a relevancy of the candidate evidence in a case that the target attribute and the decision attribute are the same; and
determining (S104) that the medical fact to be verified is correct in a case that the relevancy of the candidate evidence accords with a preset condition, and in a case where the medical fact to be verified is correct, adding the verified medical fact into a medical knowledge graph, to enrich the medical knowledge graph;
wherein after inputting (S102) the target entity, the target attribute value and the candidate evidence into the attribute decision model to obtain the decision attribute, the method further comprises: determining (S201) that the candidate evidence cannot verify that the medical fact to be verified is correct in a case that the target attribute and the decision attribute are not the same.

2. The method of claim 1, wherein the attribute decision model comprises a first natural language processing model and a first classifier; and
the inputting (S102) the target entity, the target attribute value and the candidate evidence into the attribute decision model to obtain the decision attribute comprises:
inputting the target entity, the target attribute value and the candidate evidence into the first natural language processing model to obtain a first feature vector of the target entity, the target attribute value and the candidate evidence; and
inputting the first feature vector into the first classifier to obtain the decision attribute.

3. The method of claim 2, wherein the attribute decision model is established by:
constructing the attribute decision model by using the first natural language processing model and the first classifier, wherein the first natural language processing model is a natural language processing model obtained through pre-training based on a medical corpus; and
training the constructed attribute decision model by using a plurality of first sample data, wherein each first sample data comprises a correct medical fact and supporting evidence.

4. The method of any one of claims 1-3, wherein the relevancy decision model comprises a second natural language processing model, two second classifiers, a fully connected layer, and a third classifier;
the inputting (S103) the target entity, the target attribute value and the candidate evidence into the relevancy decision model to obtain a relevancy of the candidate evidence comprises:
inputting the target entity, the target attribute value and the candidate evidence into the second natural language processing model to obtain a first layer feature vector of the target entity and the candidate evidence and a first layer feature vector of the target attribute value and the candidate evidence;
inputting the first layer feature vector of the target entity and the candidate evidence and the first layer feature vector of the target attribute value and the candidate evidence into the two second classifiers respectively, to obtain a second layer feature vector of the target entity and the candidate evidence and a second layer feature vector of the target attribute value and the candidate evidence; and
inputting the second layer feature vector of the target entity and the candidate evidence and the second layer feature vector of the target attribute value and the candidate evidence, which have been subjected to processing of the fully connected layer, into the third classifier to obtain the relevancy of the candidate evidence.

5. The method of claim 4, wherein the relevancy decision model is established by:
constructing the relevancy decision model by using the second natural language processing model, the two second classifiers, the fully connected layer and the third classifier, wherein the second natural language processing model is a natural language processing model obtained through pre-training based on a medical corpus; and
training the constructed relevancy decision model by using a plurality of second sample data, wherein each second sample data comprises a medical fact, supporting evidence and a relevancy of the medical fact and the supporting evidence.

6. The method of any one of claims 1-5, wherein the determining (S104) that the medical fact to be verified is correct in the case that the relevancy of the candidate evidence accords with the preset condition comprises:
in a case that the relevancy of at least one candidate evidence in a plurality of candidate evidence is greater than a preset threshold value, determining that the medical fact to be verified is correct, and taking the candidate evidence with a highest relevancy in the at least one candidate evidence as supporting evidence for determining that the medical fact is correct.

7. A medical fact verification apparatus (500), comprising:
a first acquisition module (501) configured for acquiring a medical fact to be verified and candidate evidence, wherein the medical fact to be verified comprises a target entity, a target attribute and a target attribute value, wherein the target attribute comprises at least one of a clinical feature, an etiology, a pathology, a therapeutic regimen, a recommended medication, a complication, and a drug effect, and the candidate evidence is retrieved from a designated medical database based on the medical fact to be verified;
a first decision module (502) configured for inputting the target entity, the target attribute value and the candidate evidence into an attribute decision model to obtain a decision attribute;
a second decision module (503) configured for inputting the target entity, the target attribute value and the candidate evidence into a relevancy decision model to obtain a relevancy of the candidate evidence in a case that the target attribute and the decision attribute are the same;
a first verification module (504) configured for determining that the medical fact to be verified is correct in a case that the relevancy of the candidate evidence accords with a preset condition, and adding the verified medical fact into a medical knowledge graph, to enrich the medical knowledge graph in a case where the medical fact to be verified is correct; and
a second verification module (601) configured for determining that the candidate evidence cannot verify that the medical fact to be verified is correct in a case that the target attribute and the decision attribute are not the same.

8. The apparatus of claim 7, wherein the attribute decision model comprises a first natural language processing model and a first classifier; and
the first decision module (502) comprises:
a feature sub-module (701) configured for inputting the target entity, the target attribute value and the candidate evidence into the first natural language processing model to obtain a first feature vector of the target entity, the target attribute value and the candidate evidence; and
an attribute decision sub-module (702) configured for inputting the first feature vector into the first classifier to obtain the decision attribute.

9. The apparatus of claim 8, wherein the attribute decision model is established by:
constructing the attribute decision model by using the first natural language processing model and the first classifier, wherein the first natural language processing model is a natural language processing model obtained through pre-training based on a medical corpus; and
training the constructed attribute decision model by using a plurality of first sample data, wherein each first sample data comprises a correct medical fact and supporting evidence.

10. The apparatus of any one of claims 7-9, wherein the relevancy decision model comprises a second natural language processing model, two second classifiers, a fully connected layer, and a third classifier; and
the second decision module (503) comprises:
a first layer feature sub-module (801) configured for inputting the target entity, the target attribute value and the candidate evidence into the second natural language processing model to obtain a first layer feature vector of the target entity and the candidate evidence and a first layer feature vector of the target attribute value and the candidate evidence;
a second layer feature sub-module (802) configured for inputting the first layer feature vector of the target entity and the candidate evidence and the first layer feature vector of the target attribute value and the candidate evidence into the two second classifiers respectively, to obtain a second layer feature vector of the target entity and the candidate evidence and a second layer feature vector of the target attribute value and the candidate evidence; and
a relevancy decision sub-module (803) configured for inputting the second layer feature vector of the target entity and the candidate evidence and the second layer feature vector of the target attribute value and the candidate evidence, which have been subjected to processing of the fully connected layer, into the third classifier to obtain the relevancy of the candidate evidence.

11. The apparatus of claim 10, wherein the relevancy decision model is established by:
constructing the relevancy decision model by using the second natural language processing model, the two second classifiers, the fully connected layer and the third classifier, wherein the second natural language processing model is a natural language processing model obtained through pre-training based on a medical corpus; and
training the constructed relevancy decision model by using a plurality of second sample data, wherein each second sample data comprises a medical fact, supporting evidence and a relevancy of the medical fact and the supporting evidence.

12. The apparatus of any one of claims 7-11, wherein the first verification module (504) comprises:
a verification sub-module (901) configured for determining that the medical fact to be verified is correct in a case that the relevancy of at least one candidate evidence in a plurality of candidate evidence is greater than a preset threshold value; and
an evidence sub-module (902) configured for taking the candidate evidence with a highest relevancy in the at least one candidate evidence as supporting evidence for determining that the medical fact is correct.

13. A non-transitory computer-readable storage medium storing computer instructions, wherein the computer instructions cause a computer to perform the method of any one of claims 1-6.

14. A computer program product comprising computer program instructions, wherein the computer program instructions cause a computer to perform the method of any one of claims 1-6.

## Patentansprüche

1. Verfahren zur Verifizierung medizinischer Sachverhalte, das von einem Computer durchgeführt wird, wobei das Verfahren umfasst:
Erfassen (S101) eines zu verifizierenden medizinischen Sachverhalts und einer Kandidatenevidenz, wobei der zu verifizierende medizinische Sachverhalt eine Zielentität, ein Zielattribut und einen Zielattributwert umfasst, wobei das Zielattribut mindestens eines der folgenden Merkmale umfasst: ein klinisches Merkmal, eine Ätiologie, eine Pathologie, ein therapeutisches Schema, eine empfohlene Medikation, eine Komplikation und eine Medikamentenwirkung, und wobei die Kandidatenevidenz aus einer bestimmten medizinischen Datenbank auf der Grundlage des zu verifizierenden medizinischen Sachverhalts abgerufen wird;
Eingeben (S102) der Zielentität, des Zielattributwerts und der Kandidatenevidenz in ein Attributentscheidungsmodell, um ein Entscheidungsattribut zu erhalten;
Eingeben (S103) der Zielentität, des Zielattributwerts und der Kandidatenevidenz in ein Relevanzentscheidungsmodell, um eine Relevanz der Kandidatenevidenz für den Fall zu erhalten, dass das Zielattribut und das Entscheidungsattribut gleich sind; und Bestimmen (S104), dass der zu verifizierende medizinische Sachverhalt korrekt ist, in einem Fall, in dem die Relevanz der Kandidatenevidenz mit einer voreingestellten Bedingung übereinstimmt, und in einem Fall, in dem der zu verifizierende medizinische Sachverhalt korrekt ist, Hinzufügen des verifizierten medizinischen Sachverhalts zu einem medizinischen Wissensgraphen, um den medizinischen Wissensgraphen zu verbessern;
wobei nach der Eingabe (S102) der Zielentität, des Zielattributwertes und der Kandidatenevidenz in das Attributentscheidungsmodell, um das Entscheidungsattribut zu erhalten, das Verfahren ferner umfasst: Bestimmen (S201), dass die Kandidatenevidenz nicht verifizieren kann, dass der zu verifizierende medizinische Sachverhalt korrekt ist, für den Fall, dass das Zielattribut und das Entscheidungsattribut nicht gleich sind.

2. Verfahren nach Anspruch 1, wobei das Attributentscheidungsmodell ein erstes natürliches Sprachverarbeitungsmodell und einen ersten Klassifikator umfasst; und das Eingeben (S102) der Zielentität, des Zielattributwerts und der Kandidatenevidenz in das Attributentscheidungsmodell, um das Entscheidungsattribut zu erhalten, umfasst:
Eingeben der Zielentität, des Zielattributwerts und der Kandidatenevidenz in das erste natürliche Sprachverarbeitungsmodell, um einen ersten Merkmalsvektor der Zielentität, des Zielattributwerts und der Kandidatenevidenz zu erhalten; und Eingeben des ersten Merkmalsvektors in den ersten Klassifikator, um das Entscheidungsattribut zu erhalten.

3. Verfahren nach Anspruch 2, wobei das Attributentscheidungsmodell erstellt wird durch:
Konstruieren des Attributentscheidungsmodells unter Verwendung des ersten natürlichen Sprachverarbeitungsmodells und des ersten Klassifikators, wobei das erste natürliche Sprachverarbeitungsmodell ein natürliches Sprachverarbeitungsmodell ist, das durch Vortraining auf der Grundlage eines medizinischen Korpus erhalten wird; und
Trainieren des konstruierten Attributentscheidungsmodells unter Verwendung einer Vielzahl von ersten Beispieldaten, wobei alle ersten Beispieldaten einen korrekten medizinischen Sachverhalt und unterstützende Beweise umfassen.

4. Verfahren nach einem der Ansprüche 1 bis 3, wobei das
Relevanzentscheidungsmodell ein zweites natürliches Sprachverarbeitungsmodell, zwei zweite Klassifikatoren, eine vollständig verbundene Schicht und einen dritten Klassifikator umfasst;
wobei das Eingeben (S103) der Zielentität, des Zielattributwertes und der Kandidatenevidenz in das Relevanzentscheidungsmodell zum Erhalt einer Relevanz der Kandidatenevidenz umfasst:
Eingeben der Zielentität, des Zielattributwerts und der Kandidatenevidenz in das zweite Modell natürlicher Sprachverarbeitung, um einen Merkmalsvektor der ersten Schicht der Zielentität und der Kandidatenevidenz und einen Merkmalsvektor der ersten Schicht des Zielattributwerts und der Kandidatenevidenz zu erhalten;
Eingeben des Merkmalsvektors der ersten Schicht der Zielentität und der Kandidatenevidenz und des Merkmalsvektors der ersten Schicht des Zielattributwertes und der Kandidatenevidenz jeweils in die zweiten Klassifikatoren, um einen Merkmalsvektor der zweiten Schicht der Zielentität und der Kandidatenevidenz und einen Merkmalsvektor der zweiten Schicht des Zielattributwertes und der Kandidatenevidenz zu erhalten; und
Eingeben des Merkmalsvektors der zweiten Schicht der Zielentität und der Kandidatenevidenz sowie des Merkmalsvektors der zweiten Schicht des Zielattributwerts und der Kandidatenevidenz, die der Verarbeitung der vollständig verbundenen Schicht unterzogen worden sind, in den dritten Klassifikator, um die Relevanz der Kandidatenevidenz zu erhalten.

5. Verfahren nach Anspruch 4, wobei das Relevanzentscheidungsmodell erstellt wird durch:
Konstruieren des Relevanzentscheidungsmodells unter Verwendung des zweiten natürlichen Sprachverarbeitungsmodells, der zwei zweiten Klassifikatoren, der vollständig verbundenen Schicht und des dritten Klassifikators, wobei das zweite natürliche Sprachverarbeitungsmodell ein natürliches Sprachverarbeitungsmodell ist, das durch Vortraining auf der Grundlage eines medizinischen Korpus erhalten wird; und
Trainieren des konstruierten Relevanzentscheidungsmodells unter Verwendung einer Vielzahl von zweiten Beispieldaten, wobei alle zweiten Beispieldaten einen medizinischen Sachverhalt, unterstützende Beweise und eine Relevanz des medizinischen Sachverhalts und der unterstützenden Beweise umfassen.

6. Verfahren nach einem der Ansprüche 1 bis 5, wobei das Bestimmen (S104), dass der zu verifizierende medizinische Sachverhalt korrekt ist, wenn die Relevanz der Kandidatenevidenz mit der voreingestellten Bedingung übereinstimmt, umfasst:
in einem Fall, in dem die Relevanz von mindestens einer Kandidatenevidenz in einer Vielzahl von Kandidatenevidenzen größer als ein voreingestellter Schwellenwert ist, Bestimmen, dass der zu verifizierende medizinische Sachverhalt korrekt ist, und Verwenden der Kandidatenevidenz mit einer höchsten Relevanz in der mindestens einen Kandidatenevidenz als unterstützender Beweis für die Bestimmung, dass der medizinische Sachverhalt korrekt ist.

7. Vorrichtung zur Verifizierung medizinischer Sachverhalte (500), umfassend;
ein erstes Erfassungsmodul (501), das zum Erfassen eines zu verifizierenden medizinischen Sachverhalts und einer Kandidatenevidenz ausgebildet ist, wobei der zu verifizierende medizinische Sachverhalt eine Zielentität, ein Zielattribut und einen Zielattributwert umfasst, wobei das Zielattribut mindestens eines der folgenden Merkmale umfasst: ein klinisches Merkmal, eine Ätiologie, eine Pathologie, ein Therapieschema, eine empfohlene Medikation, eine Komplikation und eine Arzneimittelwirkung, und wobei die Kandidatenevidenz aus einer bestimmten medizinischen Datenbank auf der Grundlage des zu verifizierenden medizinischen Sachverhalts abgerufen wird;
ein erstes Entscheidungsmodul (502), das für die Eingabe der Zielentität, des Zielattributwerts und der Kandidatenevidenz in ein Attributentscheidungsmodell dazu ausgebildet ist, ein Entscheidungsattribut zu erhalten;
ein zweites Entscheidungsmodul (503), das für die Eingabe der Zielentität, des Zielattributwerts und der Kandidatenevidenz in ein Relevanzentscheidungsmodell ausgebildet ist, um eine Relevanz der Kandidatenevidenz in dem Fall zu erhalten, dass das Zielattribut und das Entscheidungsattribut identisch sind;
ein erstes Verifizierungsmodul (504), das dazu ausgebildet ist, zu bestimmen, dass der zu verifizierende medizinische Sachverhalt in einem Fall, in dem die Relevanz der Kandidatenevidenz mit einer voreingestellten Bedingung übereinstimmt, korrekt ist, und den verifizierten medizinischen Sachverhalt in einen medizinischen Wissensgraphen einzufügen, um den medizinischen Wissensgraphen in einem Fall, in dem der zu verifizierende medizinische Sachverhalt korrekt ist, zu verbessern; und
ein zweites Verifizierungsmodul (601), das dazu ausgebildet ist, zu bestimmen, dass die Kandidatenevidenz nicht verifizieren kann, dass der zu verifizierende medizinische Sachverhalt korrekt ist, wenn das Zielattribut und das Entscheidungsattribut nicht gleich sind.

8. Vorrichtung nach Anspruch 7, wobei das Attributentscheidungsmodell ein erstes natürliches Sprachverarbeitungsmodell und einen ersten Klassifikator umfasst; und das erste Entscheidungsmodul (502) umfasst:
ein Merkmalsuntermodul (701), das für die Eingabe der Zielentität, des Zielattributwerts und der Kandidatenevidenz in das erste natürliche Sprachverarbeitungsmodell ausgebildet ist, um einen ersten Merkmalsvektor der Zielentität, des Zielattributwerts und der Kandidatenevidenz zu erhalten; und ein Attributentscheidungsuntermodul (702), das dazu ausgebildet ist, den ersten Merkmalsvektor in den ersten Klassifikator einzugeben, um das Entscheidungsattribut zu erhalten.

9. Vorrichtung nach Anspruch 8, wobei das Attributentscheidungsmodell erstellt wird durch:
Konstruieren des Attributentscheidungsmodells unter Verwendung des ersten natürlichen Sprachverarbeitungsmodells und des ersten Klassifikators, wobei das erste natürliche Sprachverarbeitungsmodell ein natürliches Sprachverarbeitungsmodell ist, das durch Vortraining auf der Grundlage eines medizinischen Korpus erhalten wird; und
Trainieren des konstruierten Attributentscheidungsmodells unter Verwendung einer Vielzahl von ersten Beispieldaten, wobei alle ersten Beispieldaten einen korrekten medizinischen Sachverhalt und unterstützende Beweise umfasst.

10. Vorrichtung nach einem der Ansprüche 7 bis 9, wobei das
Relevanzentscheidungsmodell ein zweites natürliches Sprachverarbeitungsmodell,
zwei zweite Klassifikatoren, eine vollständig verbundene Schicht und einen dritten Klassifikator umfasst; und
wobei das zweite Entscheidungsmodul (503) umfasst:
ein Merkmalsuntermodul (801) der ersten Schicht, das für die Eingabe der Zielentität, des Zielattributwerts und der Kandidatenevidenz in das zweite Modell zur natürlichen Sprachverarbeitung ausgebildet ist, um einen Merkmalsvektor der ersten Schicht der Zielentität und der Kandidatenevidenz und einen Merkmalsvektor der ersten Schicht des Zielattributwerts und der Kandidatenevidenz zu erhalten;
ein Untermodul (802) für Merkmale der zweiten Schicht, das für die Eingabe des Merkmalsvektors der ersten Schicht der Zielentität und der Kandidatenevidenz und des Merkmalsvektors der ersten Schicht des Zielattributwerts und der Kandidatenevidenz in die beiden zweiten Klassifikatoren ausgebildet ist, um einen Merkmalsvektor der zweiten Schicht der Zielentität und der Kandidatenevidenz und einen Merkmalsvektor der zweiten Schicht des Zielattributwerts und der Kandidatenevidenz zu erhalten; und
ein Relevanzentscheidungsuntermodul (803), das dazu ausgebildet ist, den Merkmalsvektor der zweiten Schicht der Zielentität und der Kandidatenevidenz und den Merkmalsvektor der zweiten Schicht des Zielattributwerts und der Kandidatenevidenz, die der Verarbeitung der vollständig verknüpften Schicht unterzogen worden sind, in den dritten Klassifikator einzugeben, um die Relevanz der Kandidatenevidenz zu erhalten.

11. Vorrichtung nach Anspruch 10, wobei das Relevanzentscheidungsmodell erstellt wird durch:
Konstruieren des Relevanzentscheidungsmodells unter Verwendung des zweiten natürlichen Sprachverarbeitungsmodells, der zwei zweiten Klassifikatoren, der vollständig verbundenen Schicht und des dritten Klassifikators, wobei das zweite natürliche Sprachverarbeitungsmodell ein natürliches Sprachverarbeitungsmodell ist, das durch Vortraining auf der Grundlage eines medizinischen Korpus erhalten wird; und
Trainieren des konstruierten Relevanzentscheidungsmodells unter Verwendung einer Vielzahl von zweiten Beispieldaten, wobei alle zweiten Beispieldaten einen medizinischen Sachverhalt, unterstützende Beweise und eine Relevanz des medizinischen Sachverhalts und der unterstützenden Beweise umfassen.

12. Vorrichtung nach einem der Ansprüche 7 bis 11, wobei das erste Verifizierungsmodul (504) umfasst:
ein Verifizierungsuntermodul (901), das dazu ausgebildet ist, zu bestimmen, dass der zu verifizierende medizinische Sachverhalt korrekt ist, wenn die Relevanz mindestens einer Kandidatenevidenz in einer Vielzahl von Kandidatenevidenzen größer ist als ein vorgegebener Schwellenwert; und
ein Evidenzuntermodul (902), das dazu ausgebildet ist, die Kandidatenevidenz mit der höchsten Relevanz in der mindestens einen Kandidatenevidenz als unterstützende Evidenz herzunehmen, um zu bestimmen, dass der medizinische Sachverhalt korrekt ist.

13. Nicht-transitorisches computerlesbares Speichermedium, das Computerbefehle speichert, wobei die Computerbefehle einen Computer veranlassen, das Verfahren nach einem der Ansprüche 1 bis 6 durchzuführen.

14. Computerprogrammprodukt, das Computerprogrammanweisungen enthält, wobei die Computerprogrammanweisungen einen Computer veranlassen, das Verfahren nach einem der Ansprüche 1 bis 6 durchzuführen.

## Revendications

1. Méthode de vérification d'un fait médical, exécutée par un ordinateur, la méthode comprenant :
acquérir (S101) un fait médical à vérifier et une preuve candidate, dans lequel le fait médical à vérifier comprend une entité cible, un attribut cible et une valeur d'attribut cible, dans lequel l'attribut cible comprend au moins l'une parmi un caractéristique clinique, une étiologie, une pathologie, un régime thérapeutique, un médicament recommandé, une complication et un effet de médicament, et la preuve candidate est extraite d'une base de données médicale désignée sur la base du fait médical à vérifier;
introduire (S102) l'entité cible, la valeur d'attribut cible et la preuve candidate dans un modèle de décision d'attribut pour obtenir un attribut de décision ;
introduire (S103) l'entité cible, la valeur d'attribut cible et la preuve candidate dans un modèle de décision de pertinence pour obtenir une pertinence de la preuve candidate dans le cas où l'attribut cible et l'attribut de décision sont identiques ; et déterminer (S104) que le fait médical à vérifier est correct dans le cas où la pertinence de la preuve candidate correspond à une condition prédéfinie, et dans le cas où le fait médical à vérifier est correct, ajouter le fait médical vérifié dans un graphe de connaissances médicales, afin d'enrichir le graphe de connaissances médicales ;
dans lequel, après avoir introduit (S102) l'entité cible, la valeur d'attribut cible et la preuve candidate dans le modèle de décision d'attribut pour obtenir l'attribut de décision, la méthode comprend en outre : déterminer (S201) que la preuve candidate ne peut pas vérifier que le fait médical à vérifier est correct dans un cas où l'attribut cible et l'attribut de décision ne sont pas identiques.

2. Méthode selon la revendication 1, dans laquelle le modèle de décision d'attribut comprend un premier modèle de traitement du langage naturel et un premier classificateur ; et
introduire (S102) l'entité cible, la valeur d'attribut cible et la preuve candidate dans le modèle de décision d'attribut pour obtenir l'attribut de décision comprend :
introduire l'entité cible, la valeur d'attribut cible et la preuve candidate dans le premier modèle de traitement du langage naturel pour obtenir un premier vecteur de caractéristiques de l'entité cible, de la valeur d'attribut cible et de la preuve candidate ; et
introduire le premier vecteur de caractéristiques dans le premier classificateur pour obtenir l'attribut de décision.

3. Méthode selon la revendication 2, dans laquelle le modèle de décision d'attribut est établi en :
construisant le modèle de décision d'attribut en utilisant le premier modèle de traitement du langage naturel et le premier classificateur, dans lequel le premier modèle de traitement du langage naturel est un modèle de traitement du langage naturel obtenu par pré-entraînement sur la base d'un corpus médical ; et
en entraînant le modèle de décision d'attribut construit en utilisant une pluralité de premiers échantillons de données, où chaque premier échantillon de données comprend un fait médical correct et une preuve de soutien.

4. Méthode selon l'une des revendications 1 à 3, dans laquelle le modèle de décision de pertinence comprend un deuxième modèle de traitement du langage naturel, deux deuxièmes classificateurs, une couche entièrement connectée et un troisième classificateur ;
introduire (S103) l'entité cible, la valeur d'attribut cible et la preuve candidate dans le modèle de décision de pertinence pour obtenir une pertinence de la preuve candidate comprend :
introduire l'entité cible, la valeur d'attribut cible et la preuve candidate dans le deuxième modèle de traitement du langage naturel pour obtenir un vecteur de caractéristique de première couche de l'entité cible et de la preuve candidate et un vecteur de caractéristique de première couche de la valeur d'attribut cible et de la preuve candidate ;
introduire le vecteur de caractéristiques de première couche de l'entité cible et de la preuve candidate et le vecteur de caractéristiques de première couche de la valeur d'attribut cible et de la preuve candidate dans les deux deuxième classificateurs respectivement, afin d'obtenir un vecteur de caractéristiques de deuxième couche de l'entité cible et de la preuve candidate et un vecteur de caractéristiques de deuxième couche de la valeur d'attribut cible et de la preuve candidate ; et
introduire le vecteur de caractéristiques de deuxième couche de l'entité cible et de la preuve candidate et le vecteur de caractéristiques de deuxième couche de la valeur d'attribut cible et de la preuve candidate, qui ont été soumis au traitement de la couche entièrement connectée, dans le troisième classificateur afin d'obtenir la pertinence de la preuve candidate.

5. Méthode selon la revendication 4, dans laquelle le modèle de décision de pertinence est établi en :
construisant le modèle de décision de pertinence en utilisant le deuxième modèle de traitement du langage naturel, les deux deuxièmes classificateurs, la couche entièrement connectée et le troisième classificateur, dans lequel le deuxième modèle de traitement du langage naturel est un modèle de traitement du langage naturel obtenu par préapprentissage sur la base d'un corpus médical ; et
en entraînant le modèle de décision de pertinence construit à l'aide d'une pluralité de deuxièmes échantillons de données, dans lequel chaque deuxième échantillon de données comprend un fait médical, une preuve de soutien et une pertinence du fait médical et de la preuve de soutien.

6. Méthode selon l'une des revendications 1 à 5, dans lequel déterminer (S104) que le fait médical à vérifier est correct dans le cas où la pertinence de la preuve candidate correspond à la condition prédéfinie comprend :
dans le cas où la pertinence d'au moins une preuve candidate dans une pluralité de preuves candidates est supérieure à une valeur seuil prédéfinie, déterminer que le fait médical à vérifier est correct, et prendre la preuve candidate ayant la pertinence la plus élevée dans au moins une preuve candidate comme preuve de soutien pour déterminer que le fait médical est correct.

7. Appareil de vérification de faits médicaux (500), comprenant ;
un premier module d'acquisition (501) configuré pour acquérir un fait médical à vérifier et une preuve candidate, dans lequel le fait médical à vérifier comprend une entité cible, un attribut cible et une valeur d'attribut cible, dans lequel l'attribut cible comprend au moins une parmi un caractéristique clinique, une étiologie, une pathologie, un régime thérapeutique, un médicament recommandé, une complication et un effet de médicament, et la preuve candidate est extraite d'une base de données médicale désignée sur la base du fait médical à vérifier;
un premier module de décision (502) configuré pour entrer l'entité cible, la valeur d'attribut cible et la preuve candidate dans un modèle de décision d'attribut afin d'obtenir un attribut de décision ;
un deuxième module de décision (503) configuré pour introduire l'entité cible, la valeur d'attribut cible et la preuve candidate dans un modèle de décision de pertinence afin d'obtenir une pertinence de la preuve candidate dans le cas où l'attribut cible et l'attribut de décision sont identiques ;
un premier module de vérification (504) conçu pour déterminer que le fait médical à vérifier est correct dans le cas où la pertinence de la preuve candidat correspond à une condition prédéfinie, et pour ajouter le fait médical vérifié à un graphe de connaissances médicales, afin d'enrichir le graphe de connaissances médicales dans le cas où le fait médical à vérifier est correct ; et
un deuxième module de vérification (601) configuré pour déterminer que la preuve candidate ne peut pas vérifier que le fait médical à vérifier est correct dans le cas où l'attribut cible et l'attribut de décision ne sont pas identiques.

8. Appareil selon la revendication 7, dans lequel le modèle de décision d'attribut comprend un premier modèle de traitement du langage naturel et un premier classificateur; et
le premier module de décision (502) comprend :
un sous-module de caractéristiques (701) configuré pour introduire l'entité cible, la valeur d'attribut cible et la preuve candidate dans le premier modèle de traitement du langage naturel afin d'obtenir un premier vecteur de caractéristiques de l'entité cible, de la valeur d'attribut cible et de la preuve candidate ; et
un sous-module de décision d'attribut (702) configuré pour introduire le premier vecteur de caractéristiques dans le premier classificateur afin d'obtenir l'attribut de décision.

9. Appareil selon la revendication 8, dans lequel le modèle de décision d'attribut est établi en :
construisant le modèle de décision d'attribut en utilisant le premier modèle de traitement du langage naturel et le premier classificateur, dans lequel le premier modèle de traitement du langage naturel est un modèle de traitement du langage naturel obtenu par préapprentissage sur la base d'un corpus médical ; et
en entraînant le modèle de décision d'attribut construit en utilisant une pluralité d'échantillons de données, dans lequel chaque premier échantillon de données comprend un fait médical correct et une preuve de soutien.

10. Appareil selon l'une des revendications 7 à 9, dans lequel le modèle de décision de pertinence comprend un deuxième modèle de traitement du langage naturel, deux deuxièmes classificateurs, une couche entièrement connectée et un troisième classificateur ; et
le deuxième module de décision (503) comprend :
un sous-module de caractéristiques de première couche (801) configuré pour entrer l'entité cible, la valeur d'attribut cible et la preuve candidate dans le deuxième modèle de traitement du langage naturel afin d'obtenir un vecteur de caractéristiques de première couche de l'entité cible et de la preuve candidate et un vecteur de caractéristiques de première couche de la valeur d'attribut cible et de la preuve candidate ;
un sous-module de caractéristiques de deuxième couche (802) conçu pour introduire le vecteur de caractéristiques de première couche de l'entité cible et de la preuve candidate et le vecteur de caractéristiques de première couche de la valeur d'attribut cible et de la preuve candidate dans les deux deuxièmes classificateurs respectivement, afin d'obtenir un vecteur de caractéristiques de deuxième couche de l'entité cible et de la preuve candidate et un vecteur de caractéristiques de deuxième couche de la valeur d'attribut cible et de la preuve candidate ; et
un sous-module de décision de pertinence (803) configuré pour introduire le vecteur de caractéristiques de deuxième couche de l'entité cible et de la preuve candidate et le vecteur de caractéristiques de deuxième couche de la valeur d'attribut cible et de la preuve candidate, qui ont été soumis au traitement de la couche entièrement connectée, dans le troisième classificateurs afin d'obtenir la pertinence de la preuve candidate.

11. Appareil selon la revendication 10, dans lequel le modèle de décision de pertinence est établi en :
construisant le modèle de décision de pertinence en utilisant le deuxième modèle de traitement du langage naturel, les deux deuxièmes classificateurs, la couche entièrement connectée et le troisième classificateur, dans lequel le deuxième modèle de traitement du langage naturel est un modèle de traitement du langage naturel obtenu par préapprentissage sur la base d'un corpus médical ; et
en entraînant le modèle de décision de pertinence construit à l'aide d'une pluralité de deuxièmes échantillons de données, chaque deuxième échantillon de données comprenant un fait médical, une preuve de soutien et une pertinence du fait médical et de la preuve de soutien.

12. Appareil selon l'une des revendications 7 à 11, dans lequel le premier module de vérification (504) comprend :
un sous-module de vérification (901) conçu pour déterminer que le fait médical à vérifier est correct dans le cas où la pertinence d'au moins une preuve candidate dans une pluralité de preuves candidates est supérieure à une valeur seuil prédéfinie ; et
un sous-module de preuve (902) configuré pour prendre la preuve candidate la plus pertinente parmi les au moins une preuve candidate comme preuve de soutien de la détermination de l'exactitude du fait médical.

13. Support de stockage non transitoire lisible par ordinateur stockant des instructions informatiques, dans lequel les instructions informatiques amènent un ordinateur à exécuter la méthode selon l'une des revendications 1 à 6.

14. Produit de programme informatique comprenant des instructions de programme informatique, dans lequel les instructions de programme informatique permettent à un ordinateur d'exécuter la méthode selon l'une des revendications 1 à 6.
